# EUROPEAN PATENT APPLICATION

(11) **EP 4 678 152 A1**
(43) Date of publication of application: **14.01.2026**
(21) Application number: 25173041.2
(22) Date of filing: 28.04.2025
(51) Int. Cl.: A61F 13/15, A61F 13/534, D04H 1/4374, D04H 1/732, A61F 13/53, A61F 13/531

(54) **PROCESS AND APPARATUS FOR FORMING A CONTINUOUS FIBROUS WEB OR A SERIES OB WEB SECTIONS THEREOF**

(30) Priority: 12.07.2024 EP 24188478
(71) Applicant: COAX Technologies S.r.l., 26010 Monte Cremasco Cremona (IT)
(72) Inventor: ZAMPOLLO, Fabio, 26013 Crema (IT)
(74) Representative: Plischke, Manfred

(57) **Abstract**

The present invention is an apparatus for and the process of manufacturing a continuous fibrous web of the air-laying type or a series of separated fibrous sections from such a web , whereby disturbances of the web formation as may create uneven fiber distribution or fiber aggregates like nits or lumps are minimized by reducing or even completely avoiding air flow leaking through the web exit opening of a forming box. A suitable leak prevention device may comprise sealing rolls or an additional suction unit positioned just downstream of the forming box.

## Description

### Field of the invention

The present invention relates to an apparatus for and the process of manufacturing a continuous fibrous web of the air-laying type or a series of separated fibrous sections from such a web , whereby disturbances of the web formation as may create uneven fiber distribution or fiber aggregates like nits or lumps are minimized by reducing or even completely avoiding air flow leaking through the web exit opening of a forming box. A suitable leak prevention device may comprise sealing rolls or an additional suction unit positioned just downstream of the forming box.

### Background

Fibrous sheet materials, particularly fibrous sheet materials for absorbing fluids, are manufactured for many uses, for example they are incorporated into absorbent articles such as disposable diapers, incontinent bed-pads and catamenial napkins as fluid absorption or fluid transmission and/or diffusion elements, for example, as absorbent cores that are intended to absorb and retain body fluids.

Dry laying and, more specifically, air laying processes are widely used to produce webs from dry fibers, which can in turn be used e.g., as sheet materials for absorbing fluids. Particularly, the air laying process refers to the formation of webs with a random fiber orientation. The fibrous sheet materials produced by air laying processes are soft, flexible and porous, and are particularly suitable for use as liquid absorbent structures in absorbent articles, such as disposable diapers, sanitary napkins, pantiliners, incontinent or bed-pads, and wipes. Air laying processes are well known in the art, as are improvements aiming at evenly distributing the fibers in the airlaid structure and/or enhancing processability.

In US5527171 (Niro) the deposition of air suspended fibers in homogeneous layers is described by using multiple rows of rotating impellers in a forming head.

Similarly, US6233787 (Dan-Web / Advanced Nonwoven S/A) shows an apparatus for uniformly distributing a disintegrated material on a fiber layer forming surface by a stirrer having impellers rotating a short distance above a collecting surface.

US20030070262 (Oerlikon) describes two air-lay stations placed one after the other serving for the dry production of a non-woven fiber web, the stations including a fiber feed duct, an air laying forming head, a perforated screen as collecting surface, and a suction box for supporting the deposition of the fibers on the screen. A separate fiber source is part of each station.

Further, WO200004232 (M&J) describes the production of a nonwoven web of fibers out of a fibrous material, such as cellulose pulp. The fibrous material is disintegrated by a hammer mill and deposited at the outlet of a forming head on an endless collection wire, thereby forming a web of fibers. Nits are extracted from the forming head via a transport fan and a second air duct. Furthermore, the nits are defibrated and returned to the forming head.

Also, WO2004106604A1 (Oerlikon) aims at avoiding nits by employing a fiber distributor in a forming head at optimizing the rotational speed of the wings of the distributor.

Such production units are typically operated in an "off-line" modus, i.e., the webs produced thereon are prepared at a wide web width for interim storage in roll- or spool-form, or boxed ("festooned") and further transported to a converting place where these are fed into a process for forming articles, such as wipes, bed-pads and the like, including the cutting to size.

Unpublished co-pending application EP24188478 (CoAx) describes an approach for forming separated web sections by depositing fibers and/or particulate material onto a collection system, such as a collection belt, comprising a plurality of air permeable regions and blocked regions which are essentially non-permeable to air.

It is a common theme in the described technologies that the web formation is performed in a forming box, to which fibers and optionally particulate materials are fed to be deposited onto a horizontal foraminous transfer belt, also referred to as "master belt", through a forming box opening. Opposite to the opening, a vacuum suction box applies vacuum, so as to support and maintain the deposition of the fibers and particles, if present. The formed web can be continuous or can be separated sections of a web, such as described in EP'478, referenced above. The web or sections thereof exit the forming box through the web exit opening as a gap between the forming box and the transfer belt and are transferred to further processing operations where a mild vacuum holds these on the transfer belt.

However, it has been found that leak air is sucked from the environment through the web exit opening into the forming box space, thereby disturbing the smooth air flow from the forming box space through the freshly formed web and the foraminous belt. This not only creates turbulences that result in an uneven fiber distribution, but also in forming fiber aggregates, such as lumps or nits.

Henceforth it is an object of the present invention to provide a solution by significantly reducing, if not completely avoiding leak air from the environment through the web exit opening into the forming box space.

Whilst EP312935 (Boscolo) describes for a co-forming system the use of "transversal walls", such as flexible lips, "that prevent turbulent air from entering" the forming box, the co-forming technology described therein with synthetic filaments being spun into the forming box provides a much stronger web as compared to the lose web of fiber/particles, allowing the transverse wall to contact the freshly formed wed or the foraminous belt.

Henceforth, the present invention aims at avoiding the disturbance through the leak air as well as damage of the web by contact with the transversal walls, such as flexible lips.

### Summary

In a first aspect, the present invention is a fibrous web forming apparatus for forming a continuous fibrous web or a series of fibrous web sections, each comprising fibers, preferably short fibers, more preferably short cellulosic fibers, and optionally particulate material. The web forming apparatus comprises a forming box and a fiber collection unit.

The forming box is separating a forming box space from the ambient environment, and is adapted to receive fibers from a fiber supply unit and optionally particulate material from a particulate material supply and to release the fibers optionally intermixed with the particulate material through a forming box outlet onto the deposition surface of the fiber collection unit.

The fiber collection unit comprises a foraminous belt moving machine-directionally from an upstream portion towards a downstream portion at a web transfer speed, and exhibits a machine direction and a cross-machine direction and a height perpendicular thereto. The fiber collection unit is adapted to transfer the web or the web sections from the web formation region downstream via a web exit opening formed between the forming box and the deposition surface of the foraminous belt of the fiber collection unit.

The apparatus further comprises a vacuum system with a first vacuum suction unit positioned opposite to the forming box outlet, relative to the foraminous belt, and adapted to support web formation in a web formation region of the forming box, and a downstream vacuum suction unit to support further web transfer downstream of the forming box.

In addition, the web forming apparatus comprises a leak air prevention device adapted to reduce leak air flow from the ambient environment through the web exit opening into the forming box. The leak air prevention device is selected from the group consisting of
- a sealing roll and a counter roll, which are positioned downstream of and in proximity to the web exit opening, preferably exhibiting a sealing roll gap distance of less than about 10 mm or less than about 5 mm, and which comprises a sealing roll drive adapted to rotated the sealing roll synchronized with the web transfer speed,
   and
- a sealing air suction unit positioned at a distance of less than about 100 mm downstream of the web exit opening and the downstream vacuum suction unit exhibiting a machine directional extension of more than 10 mm, but less than about 1000 mm and being adapted to provide a vacuum suction higher than the one of the second suction unit and lower than the one of the first suction unit.

The foraminous belt the apparatus may comprise air permeable and air impermeable regions.

The air impermeable permeable regions may extend z-directionally above the air permeable regions whereby the sealing roll exhibits a constant radius.

Alternatively, the foraminous belt may exhibit a smooth surface of the air permeable and the air impermeable regions, whereby the sealing roll exhibits a first radius and a second radius larger than the first radius and adapted to be phased with the air impermeable regions of the foraminous belt. Optionally, the fibrous web forming apparatus may comprise a first and a second forming box and further a first and a second leak air prevention device.

In another aspect, the present invention is a method for producing a continuous fibrous web or a series of fibrous web sections, each comprising fibers, preferably short fibers, more preferably short cellulosic fibers, and optionally particulate material. The method may be operated on an apparatus as described and comprises further the steps of
- forming a web or a series of web sections by
   o providing fibrous material and optionally particulate material;
   o forming a web or a series of web sections, optionally comprising particulate material, in the forming box on the web collection unit;
- feeding the web or the series of web sections through a web exit opening between the forming box and the web collection surface at a web transfer speed;
- operating the leak air prevention unit
   o by positioning the sealing roll downstream and in proximity of the web exit opening of the forming box preferably at a sealing roll gap distance of less than about 10 mm, and rotating the sealing roll at a roll surface speed matching the web transfer speed,
   ∘ or by positioning the sealing air suction unit underneath the collection belt downstream of the web exit opening at a machine directional distance of less than about 100 mm and operating the sealing air suction unit at a vacuum suction between the suction of the first vacuum suction of the deposition regions and the downstream vacuum suction unit.

Optionally, the web or web sections may be compacted simultaneously with operating the leak prevention device.

### Brief description of the Figures

Fig. 1 A to C show an apparatus according to the present invention and particular features thereof.
Fig. 2 A to C show variants of an apparatus according to the present invention.

The figures are schematic only, and not to scale. Same numerals refer to same or equivalent features or elements, single (') or multiple (", '", ...) apostrophes indicate duplicate features, such a left and right or front and back, etc. Omitting features in one figure does not imply that these cannot be combined with features of other figures.

### Detailed description

In a first aspect, the present invention relates to an apparatus and in a second aspect to the process for forming fibrous web or separated sections of such webs, comprising short fibers, such as cellulosic fibers optionally comprising particulate material.

Wood pulp fibers are a preferred starting material as may be formed by a variety of pulping processes, such as kraft pulp, sulfite pulp, thermomechanical pulp, and the like. Further, the wood fibers may be any high-average fiber length wood pulp, low-average fiber length wood pulp, or mixtures of the same. One example of suitable high-average length wood pulp fibers includes softwood fibers such as, but not limited to, northern softwood, southern softwood, redwood, red cedar, hemlock, pine (e.g., southern pines), spruce (e.g., black spruce), combinations thereof, and the like. One example of suitable low-average length wood pulp fibers includes hardwood fibers, such as, but not limited to, eucalyptus, maple, birch, aspen, and the like. The fibers may be treated so as to allow optimization of processing and or absorbency properties, The term "treated" as used herein is understood to include any means of introducing the additive to the fiber and/or fibrous matrix, but not limited to, such as coating, spraying, printing, chemical modifications, wet-end additions applications to the fibers as well as blending untreated fibers with treated fibers. Moreover, if desired, secondary fibers obtained from recycled materials may be used, such as fiber pulp from sources such as, for example, newsprint, reclaimed paperboard, and office waste, or recycled diapers, be it from factory scrap or be it post-consumer recycling.

The fibrous material may be delivered as fibrous board or in fibrous sheet form. Also bales of fibrous material may be delivered as may be opened prior to be used in the process according to the present invention. Further, "roughly graded material" may be employed, wherein fibers are present as clusters of several hundred up to several thousand fibers in the roughly graded material. It is an object of the present invention to dry lay, or air lay, such fibrous material to form fibrous webs for being converted into articles comprising such fibrous webs, such as absorbent articles, such as wipes, or pads like bed-pads, or the like. Such articles comprise further materials, typically web materials for stabilizing and/or containing the fibrous webs.

The webs may further comprise particulate material, such as superabsorbent polymeric (SAP) particles, as well known for absorbent articles, as may then be referred to as "fiber/particle" webs. Conventional air-laying processes for forming such webs are often operated in an "off-line" modus, whereby jumbo reels of the fiber/particle structure are produced on machines with a large width. Such wide roll may be cut to a lower width and rewound for being transferred to a converter, where the webs are cut to the appropriate size for being introduced into articles.

Whilst such processes allow efficient production of the fiber/particle webs, rewinding and transport imply process complexity and cost. When avoiding this by producing airlaid webs in-line on the converting process, conventional air-laying processes provide continuous webs that imply potential for contamination when being separated to the right length, and further require cut and space operation to be fed into the finished articles.

Thus, in the first aspect, the present invention is an apparatus for creating a series of separated fibrous web sections for being processed in further processing steps for forming articles comprising fibrous structures, optionally with particulate material.

**In** another aspect, the present invention is a process for forming high quality essentially endless fibrous webs, or web sections thereof, the latter being separated by regions which are free of the airlaid fibrous material and of the particulate material, if present.

In order to further explain the apparatus of and the method for executing the present invention, reference is made to the figures, which however, should not be seen to limit the present invention. In particular features as explained with reference to one figure may also be employed in the context of other figures.

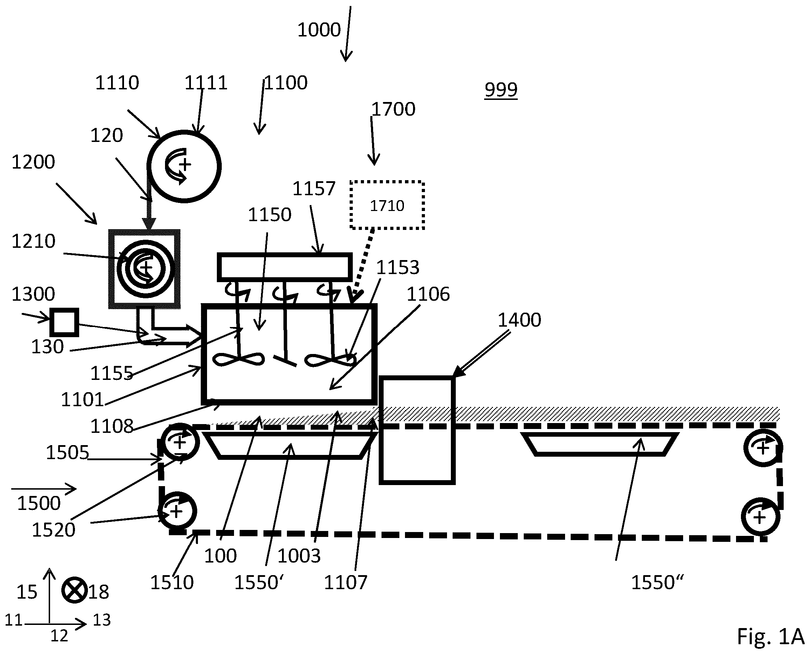

In Fig. 1A to C, exemplary apparatus 1000 for forming a fibrous web is shown, comprising a fiber supply 1110, e.g., an unwinder 1111 for delivering a fiber board 120 to a disintegration unit 1200, such as a hammer mill 1210. Alternatively or concurrently, the fibers may be delivered from other fiber supply means (not shown) such as when being delivered in bale form or from a silo or bin. Disintegrated fibers 130 are transferred, preferably pneumatically by means of air supply 1300, towards the forming box 1101 of a web forming unit 1100. As depicted, the web forming unit may comprise fiber homogenizer 1150, such as fans 1153 rotating by drive 1157 via vertical axes 1155. Optionally, and often preferably, particulate material, such as superabsorbent polymer (SAP) particles, may be fed by a particle application system 1700 from a particle supply 1710 to the web forming unit, where these may be well intermixed with fibers, whilst these are cross-directionally 18 and machine-directionally 12 homogenized. The fibers, and optionally the particulate material, are further deposited from the forming box space 1106 through the forming box outlet 1108 onto the deposition surface 1505 of a collection system 1500, forming an essentially unbonded continuous web 100, as may be also referred to as "fiber/particle" web.

In Fig. 1A, the collection system 1500 is depicted as a belt collector unit comprising a foraminous belt 1510 driven and guided by dive and guide rolls 1520. The collecting is further supported by a first suction system 1550', exhibiting a relatively strong suction, such as of an underpressure of about 5000 Pa or more versus the ambient 999. The foraminous belt 1510 and the fibrous web 100 deposited on the deposition surface 1505 thereof are moving in the machine direction 12, transferring the web from the upstream region 11 towards the web exit opening 1007, through which the web may be move towards the downstream 13 region of the apparatus and / or to further downstream processing 1800, where the web may be hold on the collection system 1500 by a downstream vacuum system 1550", exhibiting a moderate vacuum suction, such as of about 500 Pa underpressure or less versus the ambient 999.

Typically, the web exit opening 1007 is larger than the web thickness, and often also than the width of the web so as to accommodate various manufacturing options without needing to adjust the walls of the forming box 1101. However, as through the vacuum suction system 1550' a proper deposition of the fibers/particles has to be ensured, it is set to a relatively high suction, as may be 5000 Pa underpressure or more, relative to the ambient 999, resulting in significant air flow through the collection belt 1510, but which also induces a leak air flow through the web exit opening 1007, if no countermeasures were taken. This would result in turbulences at the downstream end of the forming box, which not only could result in uneven fiber and/or particle distribution, but also could create undesired fiber aggregates, such as nits or lumps.

Thus, it is an object of the present invention to minimize or even completely prevent leak air flow from the ambient 999 through the web exit opening 1007 by installing and operating a leak air prevention device 1400.

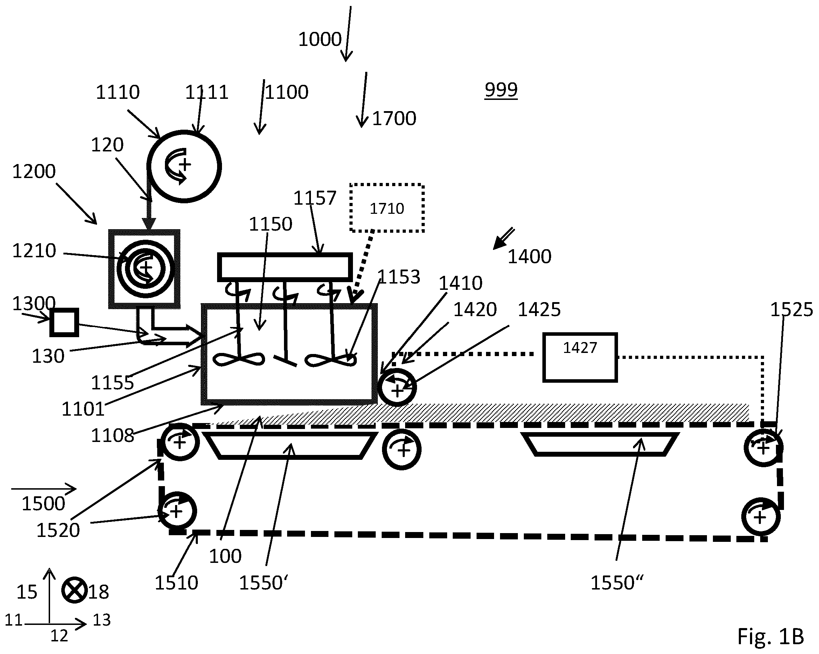

In a first execution as depicted in Fig. 1B, this is achieved by positioning a sealing roll 1420 and a counter roll 1430 in the proximity of the web exit opening 1107 and operating this at a matched speed to the web speed so as avoid any slip between the roll surface and the web surface by a sealing roll speed control 1427, exemplary triggered by the speed of the collection system 1500 and acting on the speed of the sealing roll drive 1425. Often preferred, but not necessarily, the sealing (1429) and counter (1430) roll may simultaneously compress the web 100, and optionally the sealing roll 1420 may comprise raised portions so as to allow impressing a pattern in the web. The positioning and the diameter of the sealing roll 1420 should be such that a sealing roll gap distance 1410 between the forming box and the leak roll is less than about 10 mm, or even less than about 5 mm. Optionally, the forming box may comprise a flexible skirt or a rubber lip (not shown), which may be in direct contact with the sealing roll, but not with the web surface.

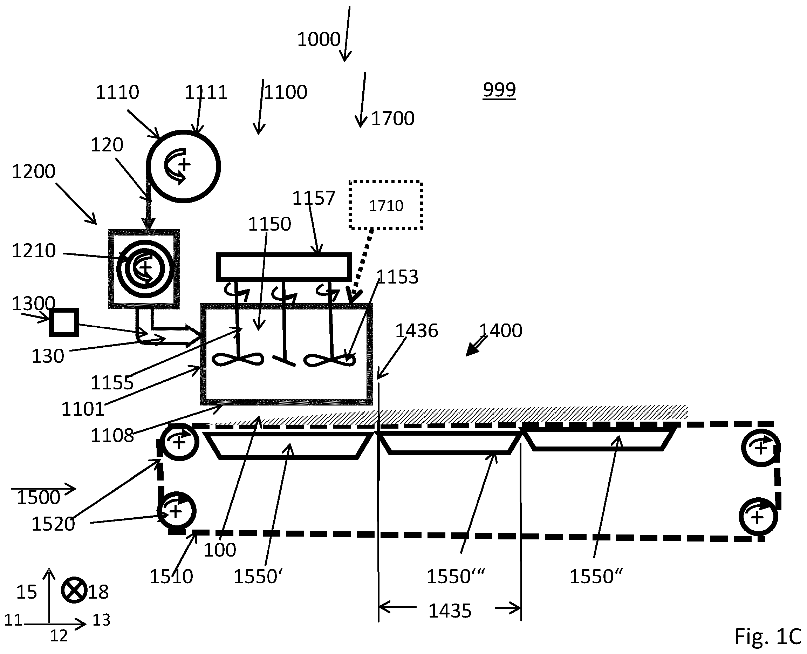

In an alternative or complementary approach, see Fig. 1C, the leak air prevention device comprises at least one additional vacuum suction system 1550‴ just downstream of the web exit opening 1007 at a distance 1436 of less than about 10 mm between the downstream end of the forming box 1101 and the upstream end of the additional vacuum suction system 1550"', which exhibits a machine directional extension 1435 of more than about 10 mm or more than about 50 mm, but typically less than about 1000 mm, or less than about 500 mm or less than about 100 mm. It is important that the vacuum suction of the additional vacuum system 1550‴ is controlled to a level lower than of the first vacuum suction system 1550', so as to not deflect fibers or particles from the forming box 1101. But it should be higher than the moderate vacuum suction of the more downstream vacuum system 1550", and may for the exemplary conditions given in the above be about 2500 Pa underpressure versus ambient 999. Optionally, the additional vacuum system 1550‴ may have a further staged of phased degree of suction, such as by implementing baffles into the vacuum suction system 1550‴. It is important that the vacuum suction of the additional vacuum suction system 1550‴ is well controlled relative to the first and to the downstream vacuum systems, 1550' and 1550", respectively, such by a vacuum control system 1428.

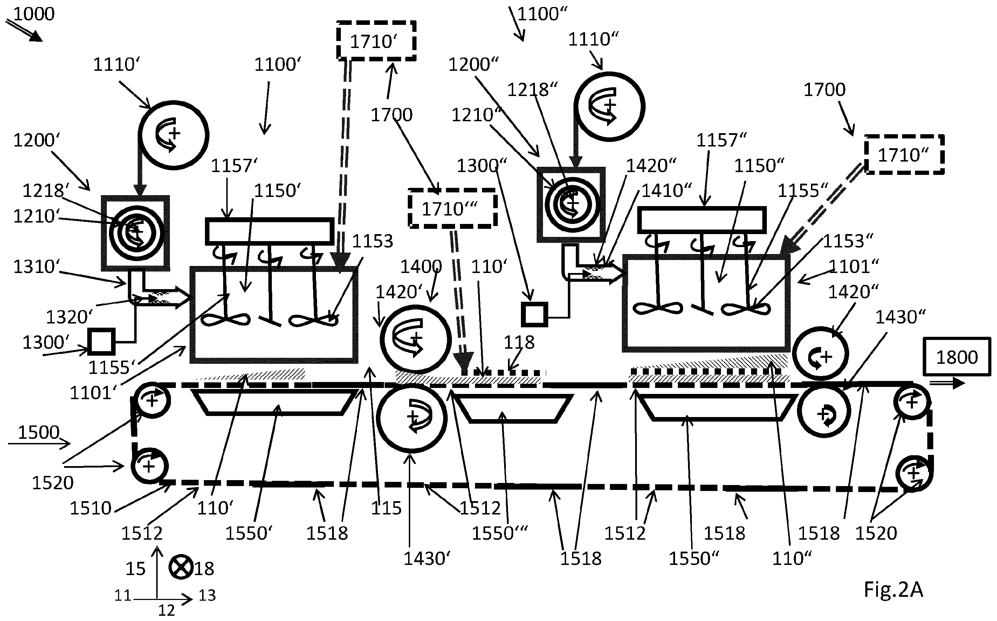

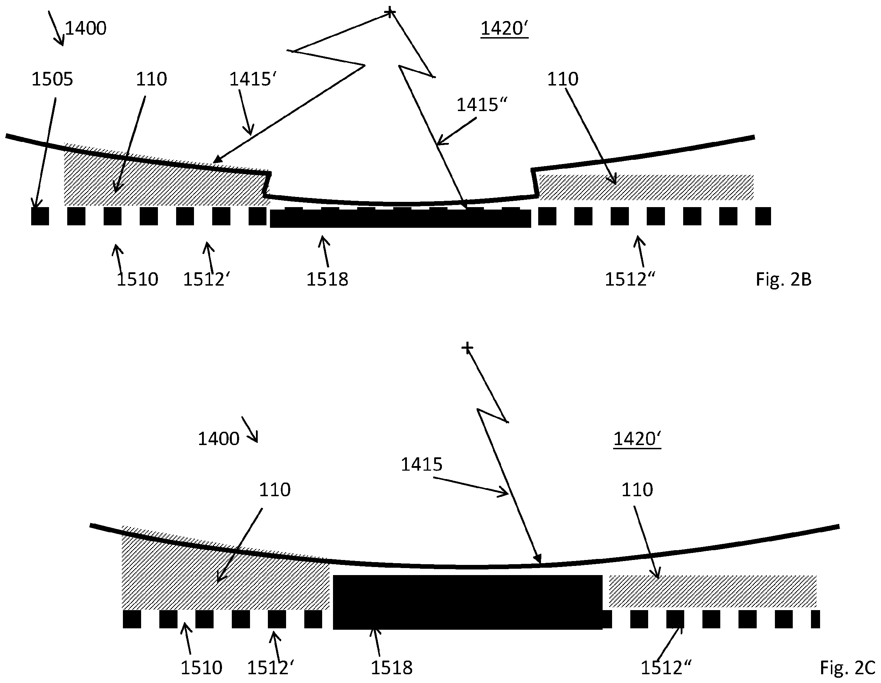

Fig. 2 depicts several variants for the present invention, which may be implemented independently or in combination.

First, instead of forming an essentially continuous web 100, the present invention may be applied to the forming of a series of web sections 110, such as by depositing the fibers and optionally particulate material onto a vacuum suction system 1550 with a collection belt 1510 comprising air permeable regions 1512 as well as air impermeable regions 1518, such that the materials are essentially only deposited onto the air permeable regions 1518, such as described in co-pending unpublished application EP254171778 (CoAx), to which express reference is made as to the forming of separated web sections 110. Thereby, the air impermeable regions 1518 of the collection belt 1510 may be executed as raised portions, relative to the air permeable regions 1512, or flat relative thereto. Referring to Fig. 2C, the sealing roll 1420 may be executed with a smooth surface and a constant radius 1415, cooperating with the raised portions of the impermeable regions 1518 of the collection belt 1510, thereby optionally reducing the thickness of the web sections 110. Alternatively, see Fig. 2B, the sealing roll 1420 may exhibit raised portions with a radius 1415" cooperating with the impermeable regions 1512, whilst the other portions with a radius 1415' may optionally compress the web sections 110 to a lower thickness.

It should be noted, that the dual functionality of sealing and compacting is optional - both for processing continuous webs 100 or a series of web sections 110.

Another optional element depicted in Fig. 2A relates to using two consecutive forming boxes for forming first web section layers 110' formed in a first forming box 1101' and second web section layers 110" in the second forming box 1101". Whilst these may be operated under varying process conditions or even with different materials, these could also be operated under identical conditions and with identical materials. As indicated, particulate material may be added in particulate material systems 1700 with a first material supply 1710' feeding into the first forming box 1101' and / or in via a second particulate material supply 1710" into the second forming box 1101". The leak air prevention device may be of the same type or different. Without implying a limitation, Fig. 2A shows a first sealing roll 1420' operating against a first counter roll 1430' which may induce compaction of the web sections 110 and smoothing of the surface just downstream of the first forming box 1101'. A second sealing roll 1420" operating against a second counter roll 1430" is positioned after the second forming box 1101", where, however, no or only very little compaction is executed so as to keep the fibrous layer 110" formed in the second forming box 1101" more lofty and open-porous than the first one 110'. The skilled person will readily realize that this can be re-applicated for the forming of continuous webs 100 from a first and a second layer of the continuous web instead of web sections 110.

A further option, particularly but not exclusively suitable for the set-up with two forming boxes refers to adding particulate material via a further particulate material addition system 1700 from a third particulate material supply 1710‴, as may sprinkle the particulate material 118 onto the web sections 110' after the first forming box and first sealing roll. In the second forming box 1101", a further layer of fibrous material 110" may be deposited.

## Claims

1. A fibrous web forming apparatus (1000)
for forming a continuous fibrous web (100) or a series of fibrous web sections (110),
each comprising fibers (130), preferably short fibers, more preferably short cellulosic fibers, and optionally particulate material (118);
said web forming apparatus (1000) comprising
- a forming box (1101) separating a forming box space (1106) from the ambient environment (999), adapted to
- receive fibers (130) from a fiber supply unit (1110) and optionally particulate material (118) from a particulate material supply (1710);
- release said fibers (130) optionally intermixed with said particulate material (118) through a forming box outlet (1108) onto the deposition surface (1505) of a fiber collection unit (1500),
- said fiber collection unit (1500)
comprising a foraminous belt (1510) moving machine-directionally from an upstream portion (11) towards a downstream portion (13) at a web transfer speed, and exhibiting a machine direction (12) and a cross-machine direction (18) and a height (15) perpendicular thereto,
and being adapted to transfer said web (100) or said web sections (110) from said web formation region (1103) downstream (13) via a web exit opening (1107) formed between said forming box (1101) and said deposition surface (1505) foraminous belt (1510);
- a vacuum system (1500) comprising
- a first vacuum suction unit (1550') positioned opposite to said forming box outlet (1108), relative to said foraminous belt (1510), and adapted to support web formation in a web formation region (1103) of said forming box (1101);
- a downstream vacuum suction unit (1550") to support further web transfer downstream (13) of said forming box (1101),
**characterized in that**
said web forming apparatus (1000) further comprises a leak air prevention device (1400) adapted to reduce leak air flow from said ambient environment (999) through said web exit opening (1107) into said forming box (1101)
said leak air prevention device (1400) being selected from the group consisting of
- a sealing roll (1420) and a counter roll (1430),
o positioned downstream (13) of and in proximity to said web exit opening (1107), preferably at a sealing roll gap distance of less than about 10 mm,
∘ comprising a sealing roll drive (1425) adapted to rotated said sealing roll (1420) synchronized with said web transfer speed;
- a sealing air suction unit (1550‴)
∘ positioned between said web exit opening (1107) and said downstream vacuum suction unit (1500") preferably at a distance of less than about 100 mm, more preferably less than about 50 mm, and
∘ exhibiting a machine directional extension (1435) of more than 10 mm, but less than about 1000 mm;
∘ and being adapted to provide a vacuum suction higher than the one of said second suction unit (1500") and lower than the one of said first suction unit (1550').

2. A fibrous web forming apparatus (1000) according to claim 1,
wherein said foraminous belt (1510) of said collection unit (1500) comprises air permeable (1512) and air impermeable (1518) regions.

3. A fibrous web forming apparatus according to claim 2, wherein said air impermeable regions (1518) of said foraminous belt (1510) are extending z-directionally above said air permeable regions (1512), and wherein said sealing roll (1420) exhibits a constant radius (1415).

4. A fibrous web forming apparatus according to claim 3, wherein said foraminous belt (1510) exhibit a smooth surface of said air permeable (1512) and said air impermeable (1518) regions, and wherein said sealing roll (1420) exhibits a first radius (1415') and a second radius (1415") larger than said first radius (1415') and adapted to be phased with said air impermeable regions (1518) of said foraminous belt (1510).

5. A fibrous web forming apparatus according to claim 1,
comprising a first (1101') and a second (1101") forming box
and further a first (1400') and a second (1400") leak air prevention device.

6. A method for producing a continuous fibrous web (100) or a series of fibrous web sections (110), each comprising fibers (130), preferably short fibers, more preferably short cellulosic fibers, and optionally particulate material (118),
said process comprising the steps of
- providing an apparatus according to any of claims 1 to 5;
- forming a web (100) or a series of web sections (110) by
∘ providing fibrous material (130) and optionally particulate material (118);
∘ forming a web (100) or a series of web sections (110), optionally comprising particulate material (118), in said forming box (1101) on said web collection unit (1500);
- feeding said web (100) or said series of web sections (110) through a web exit opening (1107) between said forming box and said web collection surface (1505) at a web transfer speed;
- operating said leak air prevention unit (1400)
∘ by positioning said sealing roll (1420) downstream and in proximity of said web exit opening (1107) of said forming box (1101) at a sealing roll gap distance (1410) of less than about 5 mm, and rotating said sealing roll (1420) at a roll surface speed matching said web transfer speed
∘ or by positioning said sealing air suction unit (1500‴) underneath said collection belt (1510) downstream of said web exit opening (1107) at a machine directional distance of less than about 100 mm and operating said sealing air suction unit (1500‴) at a vacuum suction between the suction of said first vacuum suction (1550') of said deposition regions (1103) and said downstream vacuum suction unit (1550").

7. A method for producing a continuous fibrous web (100) or a series of fibrous web sections (110) according to claim 6,
wherein simultaneously with said operating of said leak air prevention device (1400) compacting said fibrous web (100) or web section (110).
